(19) Europäisches Patentamt
European Patent Office
Office européen des brevets

(11) Veröffentlichungsnummer: **0 068 370**
**A1**

(12) # EUROPÄISCHE PATENTANMELDUNG

(21) Anmeldenummer: **82105395.6**

(22) Anmeldetag: **19.06.82**

(51) Int. Cl.³: **C 07 D 313/12**, C 07 D 491/044, C 07 D 493/04, C 07 D 495/04 // A61K31/335 ,(C07D491/044, 313/00, 221/00),(C07D493/04, 313/00, 307/00),(C07D495/04, 333/00, 313/00)

(30) Priorität: **27.06.81 DE 3125374**

(43) Veröffentlichungstag der Anmeldung: **05.01.83 Patentblatt 83/1**

(84) Benannte Vertragsstaaten: **AT CH DE IT LI LU NL SE**

(71) Anmelder: **HOECHST AKTIENGESELLSCHAFT, Postfach 80 03 20, D-6230 Frankfurt/Main 80 (DE)**

(72) Erfinder: **Militzer, Hans, Dr., Königsteiner Strasse 15, D-6238 Hofheim am Taunus (DE)**

(54) **Verfahren zur Herstellung von Heteroaryl- und Dibenzoxepinalkansäuren.**

(57) Herstellung von Heteroarylbenzoxepinalkansäuren und 6,11-Dihydro-11-oxodibenz[b, e]oxepinalkansäuren der Formel I

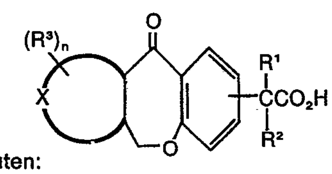

worin bedeuten:

X zusammen mit den Kohlenstoffatomen, an die es gebunden ist, einen Benzolring oder einen 5- oder 6gliedrigen Heteroarylring, der 1 oder 2 Atome Sauerstoff, Stickstoff oder Schwefel enthält; $R^1$ und $R^2$ jeweils Wasserstoff oder eine Methylgruppe;

$R^3$ Alkyl oder Alkoxy mit 1–4 Kohlenstoffatomen, Halogen oder die Trifluormethylgruppe und

n die Zahl 0, 1, 2 oder 3,

dadurch gekennzeichnet, dass man eine Verbindung der Formel II

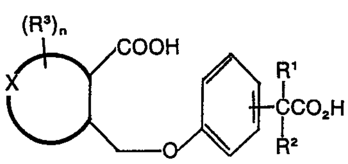

worin X, $R^1$, $R^2$, $R^3$ und n die oben genannte Bedeutung haben, in Gegenwart eines gegebenenfalls halogenierten aliphatischen Carbonsäureanhydrids oder Carbonsäurehalogenids und katalytischen Mengen einer starken Säure auf 50–150 °C erhitzt.

EP 0 068 370 A1

Verfahren zur Herstellung von Heteroaryl- und Dibenzoxepinalkansäuren

Die vorliegende Erfindung betrifft ein Verfahren zur Herstellung von Heteroarylbenzoxepinalkansäuren bzw. 6,11-Dihydro-11-oxo-dibenz/b,e/oxepinalkansäuren der Formel I

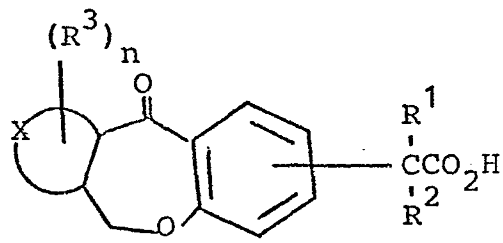

worin bedeuten:

X zusammen mit den Kohlenstoffatomen, an die es gebunden ist, einen Benzolring oder einen 5- oder 6-gliedrigen Heteroarylring, der 1 oder 2 Atome Sauerstoff, Stickstoff oder Schwefel enthält;

$R^1$ und $R^2$ jeweils Wasserstoff oder eine Methylgruppe;

$R^3$ Alkyl oder Alkoxy mit 1 - 4 Kohlenstoffatomen, Halogen oder die Trifluormethylgruppe und

n die Zahl 0, 1, 2 oder 3,

das dadurch gekennzeichnet ist, daß man eine Verbindung der Formel II

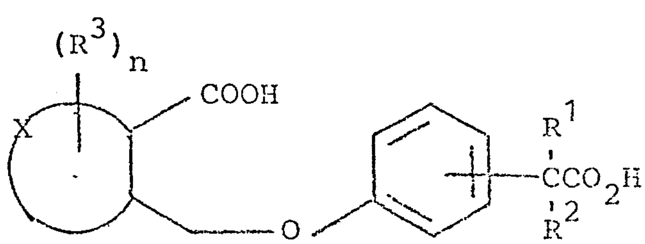

worin X, $R^1$, $R^2$, $R^3$ und n die oben genannte Bedeutung haben, in Gegenwart eines aliphatischen $C_2$-$C_5$-Carbonsäureanhydrids

bzw. $C_2$-$C_5$-Carbonsäurehalogenids und katalytischen Mengen einer starken Säure auf 50 - 150°C erhitzt.

Bevorzugt können nach dem erfindungsgemäßen Verfahren Verbindungen der Formel I hergestellt werden, worin die Substituenten die folgende Bedeutung haben:

X bedeutet zusammen mit den Kohlenstoffatomen, an die es gebunden ist, einen Benzolring, der mit F, Cl, Br, $CF_3$, $CH_3$ oder $OCH_3$ jeweils einfach substituiert sein kann, oder einen der folgenden Heteroarylreste

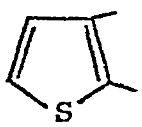 , 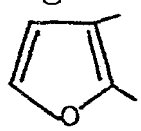 , 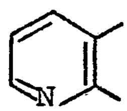 , 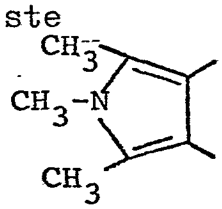 ,

$R^1$ die Methylgruppe und $R^2$ Wasserstoff.

Die Verbindungen der Formel I und Verfahren zu ihrer Herstellung sind bereits beschrieben in den DE-OS 24 42 060, 26 00 768 und 26 37 110.

In dem Verfahren gemäß den DE-OS 24 42 060 und dem Verfahren A der DE-OS 26 37 110 wird eine gegebenenfalls substituierte Carboxybenzyloxyphenylessigsäure durch Behandlung mit einem dehydrierenden Mittel, wie Polyphosphorsäure, Äthanol, Phosphorpentoxid oder Schwefelsäure mit oder ohne Lösungsmittel, wie Tetramethylensulfon oder Essigsäure bei 50°C bis 125°C 15 Minuten zyklisiert, wobei eine 6,11-Dihydro-11-oxodibenz-[b,e]oxepinessigsäure entsteht.

Das Verfahren gemäß der DE-OS 26 00 768 bzw. der DE-OS 26 37 110 geht von der Dicarbonsäure der Formel II aus, die zunächst mit einem Halogenierungsmittel wie z.B. Thionylhalogenid oder Phosphorpentahalogenid in das entsprechende Dicarbonsäuredihalogenid überführt wird. Das Dicarbonsäuredihalogenid wird dann unter Friedel-Crafts-Bedingungen oder thermisch zyklisiert und anschließend in bekannter Weise hydrolysiert zu einer Verbindung der Formel I. Die beschriebenen Verfahren ergeben zwar gute Ausbeuten, erfordern jedoch im Anschluß an die Umsetzungen zum Teil aufwendige Aufarbeitung und Reinigung der Rohprodukte. Beide Verfahren haben zudem den Nachteil, daß große Mengen belastete Abwässer abgeführt werden müssen. So fallen bei dem zuerst genannten Verfahren pro Mol Endprodukt 7,9 Mol Phosphorsäure an, bei dem zweiten Verfahren z.B. 4 Mol HCl und 2 Mol schweflige Säure pro Mol Endprodukt.

Überraschenderweise wurde nun gefunden, daß die Zyklisierung der Dicarbonsäure der Formel II mit einem gegebenenfalls halogenierten aliphatischen Carbonsäureanhydrid oder Carbonsäurehalogenid in Gegenwart von katalytischen Mengen einer starken Säure zu guten Ausbeuten der Verbindung der Formel I führt, wobei eine aufwendige Aufarbeitung des Endprodukts und Abwasserbelastung entfallen bzw. erheblich reduziert werden.
Als Säure kommt z.B. infrage Phosphorsäure, Toluolsulfonsäure oder Schwefelsäure.

Bevorzugt wird das erfindungsgemäße Verfahren mit 1 bis 1,5 Mol eines aliphatischen $C_2$-$C_5$-Carbonsäureanhydrids, z.B. Acetanhydrid, oder 1 bis 1,5 Mol eines aliphatischen $C_2$-$C_5$-Carbonsäurechlorids, z.B. Acetylchlorid in Gegenwart von 1 - 10 Mol-% 85 %iger Phosphorsäure in einem inerten Lösungsmittel, z.B. einem aromatischen Kohlenwasserstoff wie Toluol, Xylol oder Chlorbenzol oder in einem Überschuß des betreffenden Carbonsäureanhydrids bzw. Carbonsäurechlorids, bei Temperaturen von 50 bis 150°C, insbesondere bei 100 - 140°C durchgeführt.

Zweckmäßigerweise wird die während der Reaktion entstehende Carbonsäure, z.B. Essigsäure, aus der Reaktionsmischung abdestilliert, um die Reaktionszeit abzukürzen.

Die Ausbeute kann auch erhöht bzw. die Aufarbeitung der Rohstoffe vereinfacht werden, wenn bei der erfindungsgemäßen Zyklisierungsreaktion inerte Trägerstoffe wie z.B. Kieselgur, Alkalialuminiumsilikate oder Aktivkohle zugegen sind. In diesem Fall lagern sich die Phosphorsäure und Neben- bzw. Zersetzungsprodukte, die zum Teil stark gefärbt sind, an dem Trägermaterial ab. Der feste Rückstand, der das Trägermaterial, adsorbierte Phosphorsäure und die Neben- bzw. Zersetzungsprodukte enthält, kann leicht abfiltriert werden und verbrannt bzw. in einer Deponie gelagert werden, so daß es nicht zu einer Abwasserbelastung kommt.

Die nach dem erfindungsgemäßen Verfahren hergestellten Verbindungen haben antiphlogistische und analgetische Eigenschaften und können daher als Arzneimittel verwendet werden.

Außer den in den Beispielen genannten Verbindungen können insbesondere auch die folgenden Verbindungen nach dem erfindungsgemäßen Verfahren hergestellt werden.

6,11-Dihydro-8-methyl-11-oxodibenz[b,e]oxepin-2-essigsäure,
6,11-Dihydro-8-methoxy-11-oxodibenz[b,e]oxepin-2-essigsäure,
6,11-Dihydro-9-fluor-11-oxo-dibenz[b,e]oxepin-2-essigsäure,
4,10-Dihydro-10-oxothieno[3,2-c] [1]benzoxepin-8-essigsäure,
4,10-Dihydro-10-oxofurano[3,2-c] [1]benzoxepin-8-essigsäure,
4,10-Dihydro-1,2,3-trimethyl-10-oxopyrrolo-[3,4-c] [1]benzoxepin-8-essigsäure,
5,11-Dihydro-11-oxopyrimido[4,5-c] [1]benzoxepin-9-essigsäure.

Beispiele:

Beispiel 1

14,3 g (0,05 Mol) 4-(2-Carboxyphenoxy)-phenylessigsäure werden in 50 ml Xylol suspendiert und mit 7,3 g (0,07 Mol) Acetanhydrid versetzt. Man erhitzt zum Rückfluß und tropft 0,34 g (0,003 Mol) 85 %ige Phosphorsäure zu. Man rührt 5 Stunden unter Rückfluß nach, dekantiert die Lösung ab, kühlt auf Raumtemperatur ab und saugt das ausgefallene Produkt ab, das anschließend im Vakuum bei 60°C getrocknet wird.
Die Ausbeute beträgt 10,2 g 6,11-Dihydro-11-oxodibenz/b,e7-oxepin-2-essigsäure (76 d.Th.). Schmelzpunkt: 137°C.

Beispiel 2

14,3 g (0,05 Mol) 4-(2-Carboxyphenoxy)-phenylessigsäure werden mit 54,3 g Acetanhydrid versetzt. Nach dem Erhitzen auf 130°C werden 1,71 g (0,015 Mol) 85 %ige Phosphorsäure zugetropft. Die Mischung wird 20 Minuten unter Rückfluß erhitzt. Dann werden 100 ml Wasser zugetropft. Nach dem Abkühlen auf Raumtemperatur wird das auskristallisierte Produkt abgesaugt, mit Wasser gewaschen und im Vakuum bei 60°C getrocknet.
Die Ausbeute beträgt 9,8 g des im Beispiel 1 genannten Endproduktes (72 % d.Th.). Schmelzpunkt: 133 - 135°C.

Beispiel 3

14,3 g (0,05 Mol) 4-(2-Carboxyphenoxy)-phenylessigsäure, 50 ml Toluol, 4,9 g (0,026 Mol) Acetylchlorid und 0,5 g (0,0044 Mol) 85 %ige Phosphorsäure werden 3 1/2 Stunden unter Rückfluß erhitzt. Es wird mit Aktivkohle filtriert und auf Raumtemperatur abgekühlt. Das auskristallisierte Produkt wird abgesaugt, mit Toluol gewaschen und im Vakuum bei 60°C getrocknet.
Die Ausbeute beträgt 10,1 g des im Beispiel 1 genannten Endproduktes (75,4 % d.Th.). Schmelzpunkt: 134 - 136°C.

Beispiel 4

57,2 g (0,2 Mol) 4-(2-Carboxyphenoxy)-phenylessigsäure, 1,6 g Dicalite, 200 ml Xylol, 26,0 g (0,25 Mol) Acetanhydrid und 2,05 g (0,018 Mol) 85 %ige Phosphorsäure werden unter Rühren 1 Stunde unter Rückfluß erhitzt. Dann werden innerhalb von 30 Minuten 80 ml Essigsäure/Xylol-Gemisch abdestilliert und 1,6 g Aktivkohle zugegeben. Man filtriert die Mischung und kühlt das Filtrat auf Raumtemperatur ab. Das abdestillierte Produkt wird abgesaugt, mit Xylol gewaschen und im Vakuum bei 60°C getrocknet.
Die Ausbeute beträgt 42,9 g des in Beispiel 1 angegebenen Endproduktes (80,0 % d.Th.). Schmelzpunkt: 137°C.

Beispiel 5

57,2 g (0,2 Mol) 4-(2-Carboxyphenoxy)-phenylessigsäure, 1,6 g Dicalite, 200 ml Xylol, 19,8 g (0,25 Mol) Acetylchlorid und 2,05 g (0,018 Mol) 85 %ige Phosphorsäure werden unter Rühren 1 Stunde unter Rückfluß erhitzt. Dann werden innerhalb von 30 Minuten 80 ml Essigsäure/Xylol-Gemisch abdestilliert und 1,6 g Aktivkohle zugegeben. Man filtriert die Mischung und kühlt das Filtrat auf Raumtemperatur ab. Das auskristallisierte Produkt wird abgesaugt, mit Xylol gewaschen und im Vakuum bei 60°C getrocknet.
Die Ausbeute beträgt 40,4 g des in Beispiel 1 genannten Endproduktes (73,4 % d.Th.). Schmelzpunkt: 134 - 136°C.

Beispiel 6

2,0 g 2-Carboxy-3-[α-(p-carboxymethyl)-phenoxymethyl]-thiophen werden mit 15 ml Xylol, 1,1 ml Acetanhydrid, 0,06 g Dicalite und 0,08 ml 85 proz. Phosphorsäure versetzt und 3 Stunden unter Rückfluß erhitzt. Man dekantiert vom Rückstand ab und läßt das Produkt über Nacht bei Raumtemperatur auskristallisieren. Es wird abgesaugt, aus Aceton/Wasser nungelöst und im Vakuum bei 60°C getrocknet. Man erhält 0,9 g 4,10-Dihydro-10-oxothieno[3,2-c][1]-benzoxepin-8-essigsäure.
Schmelzpunkt: 162 - 164°C.

Patentansprüche:

1. Verfahren zur Herstellung von Heteroarylbenzoxepinalkansäuren und 6,11-Dihydro-11-oxodibenz/b,e/oxepinalkansäuren der Formel I

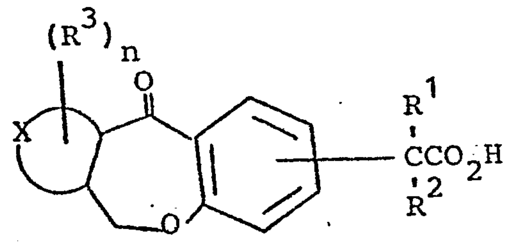

worin bedeuten:

X zusammen mit den Kohlenstoffatomen, an die es gebunden ist, einen Benzolring oder einen 5- oder 6-gliedrigen Heteroarylring, der 1 oder 2 Atome Sauerstoff, Stickstoff oder Schwefel enthält; $R^1$ und $R^2$ jeweils Wasserstoff oder eine Methylgruppe;

$R^3$ Alkyl oder Alkoxy mit 1 - 4 Kohlenstoffatomen, Halogen oder die Trifluormethylgruppe und

n die Zahl 0, 1, 2 oder 3,

dadurch gekennzeichnet, daß man eine Verbindung der Formel II

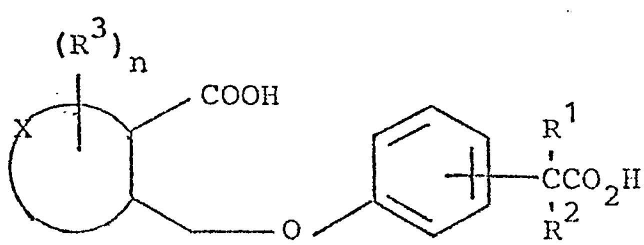

worin X, $R^1$, $R^2$, $R^3$ und n die oben genannte Bedeutung haben, in Gegenwart eines gegebenenfalls halogenierten aliphatischen Carbonsäureanhydrids oder Carbonsäurehalogenids und katalytischen Mengen einer starken Säure auf 50 - 150°C erhitzt.

2. Verfahren gemäß Anspruch 1, dadurch gekennzeichnet, daß 1 Mol einer Verbindung der Formel II mit 1 - 1,5 Mol eines aliphatischen $C_2$-$C_5$-Carbonsäureanhydrids oder aliphatischen $C_2$-$C_5$-Carbonsäurehalogenids in Gegenwart von 1 - 10 Mol-% konzentrierter Phosphorsäure und in einem inerten organischen Lösungsmittel erhitzt wird.

3. Verfahren gemäß Anspruch 1 und 2, dadurch gekennzeichnet, daß ohne Lösungsmittel, jedoch in Gegenwart eines Überschusses an Carbonsäureanhydrid oder -halogenid erhitzt wird.

4. Verfahren gemäß Anspruch 1 und 2, dadurch gekennzeichnet, daß 1 Mol einer Verbindung der Formel II mit 1 - 1,5 Mol Acetanhydrid oder 1 - 1,5 Mol Acetylchlorid in Gegenwart von 1 - 10 Mol-% konzentrierter Phosphorsäure und in einen aromatischen Kohlenwasserstoff erhitzt wird.

5. Verfahren gemäß Anspruch 1 - 4, dadurch gekennzeichnet, daß die Reaktionsmischung auf 100 - 140°C erhitzt wird.

6. Verfahren gemäß Anspruch 1 - 4, dadurch gekennzeichnet, daß die Reaktionsmischung in Gegenwart eines inerten Trägermaterials erhitzt wird.

7. Verfahren gemäß Anspruch 6, dadurch gekennzeichnet, daß als Trägermaterial Kieselgur, ein Alkali-Aluminiumsilikat oder Aktivkohle verwendet wird.

Europäisches Patentamt

**EUROPÄISCHER RECHERCHENBERICHT** 0068370

Nummer der Anmeldung

EP 82 10 5395

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | DE-A-2 435 613 (DAIICHI)<br>* das ganze Dokument; insbesondere Beispiele 1-19 ,21; Seite 6, zweiter Absatz *<br>--- | 1 | C 07 D 313/12<br>C 07 D 491/044<br>C 07 D 493/04<br>C 07 D 495/04 //<br>A 61 K 31/335<br>(C 07 D 491/044<br>C 07 D 313/00<br>C 07 D 221/00 )<br>(C 07 D 493/04<br>C 07 D 313/00<br>C 07 D 307/00 )<br>(C 07 D 495/04<br>C 07 D 333/00<br>C 07 D 313/00 ) |
| D,A | DE-A-2 600 768 (HOECHST)<br>* das ganze Dokument *<br>--- | 1 | |
| D,A | DE-A-2 442 060 (HOECHST)<br>* das ganze Dokument *<br>--- | 1 | |
| D,A | DE-A-2 637 110 (HOECHST)<br>* das ganze Dokument *<br>--- | 1 | |
| P,A | US-A-4 282 365 (J. ROKACH)<br>* das ganze Dokument; insbesondere Beispiele 1,8; Spalten 3-4,7-8 *<br>----- | | |

RECHERCHIERTE SACHGEBIETE (Int. Cl. 3)

C 07 D 313/00
C 07 D 491/00
C 07 D 493/00
C 07 D 495/00

Der vorliegende Recherchenbericht wurde für alle Patentansprüche erstellt.

| Recherchenort | Abschlußdatum der Recherche | Prüfer |
|---|---|---|
| DEN HAAG | 28-09-1982 | NUYTS A.M.K.A. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument